# EUROPÄISCHE PATENTANMELDUNG

(11) **EP 3 045 140 A1**
(43) Veröffentlichungstag der Anmeldung: **20.07.2016**
(21) Anmeldenummer: 15202003.8
(22) Anmeldetag: 22.12.2015
(51) Int. Cl.: A61B 34/37

(54) **VERFAHREN ZUR AUSRICHTUNG EINES MEHRACHSIGEN MANIPULATORS MIT EINEM EINGABEGERÄT**

(30) Priorität: 14.01.2015 DE 102015200428
(71) Anmelder: KUKA Roboter GmbH, 86165 Augsburg (DE)
(72) Erfinder: LOHMEIER, Sebastian, 80639 München (DE); VON TIESENHAUSEN, Cyrill, 86150 Augsburg (DE)
(74) Vertreter: Mader, Joachim

(57) **Zusammenfassung**

Es wird ein Verfahren bereitgestellt zur Ausrichtung eines mehrachsigen Manipulators mit einem Eingabegerät, welches der Steuerung des Manipulators dient, aufweisend die Schritte eines Ausführens einer oder mehrerer Referenzbewegungen mit dem Eingabegerät, eines Ausführens einer oder mehrerer Referenzbewegungen mit dem Manipulator, eines Erfassens der ausgeführten Referenzbewegungen, eines Berechnens einer Transformationsmatrix basierend auf den erfassten Referenzbewegungen und eines Anwendens der berechneten Transformationsmatrix zur Ausrichtung der Bewegungen des Eingabegeräts mit dem Manipulator.

## Beschreibung

### 1. Technischer Bereich

Die vorliegende Erfindung betrifft allgemein ein Verfahren zur Ausrichtung eines mehrachsigen Manipulators mit einem Eingabegerät und insbesondere ein vereinfachtes Ausrichten des Referenz-Koordinatensystems eines Eingabegeräts zum Koordinatensystem eines damit zu bedienenden Manipulators.

### 2. Technischer Hintergrund

Manipulatoren, wie insbesondere Gelenkarmroboter, werden für verschiedene Arbeitsprozesse in beispielsweise der Montage oder Fertigung im industriellen Umfeld eingesetzt. Weiterhin finden Manipulatoren Anwendung im medizinischen, insbesondere dem chirurgischen Umfeld zur Durchführung von und Unterstützung bei Operationen, insbesondere bei Tätigkeiten, die eine besonders akkurate Handhabung von Werkzeug erfordern.

Manipulatoren können dabei beweglich oder an einem festen Ort eingesetzt werden. Der Manipulator ist dabei vorzugsweise ein dreidimensional beweglicher Gelenkarmroboter, welcher aus mehreren Dreh- und/oder Schubgelenken aufgebaut ist. Üblicherweise sind die Dreh- bzw. Schubgelenke mit Armgliedern verbunden, und jedes Dreh- bzw. Schubgelenk ist durch eine Gelenkachse ausgezeichnet. Ein Robotersystem umfasst einen oder mehrere Manipulatoren, eine Steuereinrichtung und ein Eingabegerät.

Es sind Robotersysteme in der Chirurgie oder interventionellen Radiologie bekannt, bei denen die Eingabegeräte an einer Bedienkonsole befestigt sind. Die relative Ausrichtung der Eingabegeräte ist festgelegt und kann nicht geändert werden. Dies erschwert die Nutzung durch den Arzt, da die Bewegungsfreiheit und der Zugang zum Patienten erschwert wird.
Weiter sind Systeme bekannt, bei denen ein flexibler Katheter durch einen Roboter vorgeschoben und gesteuert wird. Das Eingabegerät ist bei diesem System räumlich vom Roboter getrennt. Die Ausrichtung des Eingabegerätes ist jeweils relativ zur Spitze des Instrumentes, wobei das Instrument am Ende des Roboterarms angebracht ist.

Schließlich sind Robotersysteme bekannt, bei denen Eingabegeräte räumlich getrennt vom Arbeitsplatz des Manipulators aufgestellt sind, sogenannte Telemanipulationssysteme. Die Eingabegeräte sind an einer Bedienkonsole befestigt, wobei die Ausrichtung der Eingabegeräte relativ zu einer auf einem Bildschirm dargestellten kameragestützten, z.B. endoskopischen Bilddarstellung ist, deren Ausrichtung zu dem Manipulator bzw. den Manipulatoren des Robotersystems festgelegt ist.

Den oben genannten Systemen ist allen gleich, dass das Eingabegerät räumlich getrennt von dem Manipulator ist oder die Ausrichtung des Eingabegeräts relativ zu dem zu bedienenden Manipulator vorgegeben ist. Dies hat den Nachteil, dass die Ausrichtung der Eingabegeräte während der Verwendung z.B. intraoperativ nicht ohne weiteres verändert werden kann. Zwar wäre es denkbar die Position des Eingabegeräts zu ändern, dadurch würde sich aber auch die Position des Eingabegeräts zum Manipulator ändern, wodurch die Steuerung des Robotersystems nicht mehr intuitiv durchgeführt werden kann. Beispielsweise sind die Eingabegeräte üblicherweise so mit dem zu bedienenden Manipulator ausgerichtet, dass eine Vorwärtsbewegung eines z.B. Joysticks, zu einer entsprechenden Vorwärtsbewegung des Manipulators in dieselbe Richtung führt. Dreht man das Eingabegerät um z.B. 90° ist diese für den Bediener intuitiv einfache Kopplung nicht mehr gegeben.

Ein weiterer Nachteil der oben genannten Systeme ist, dass ein gleichzeitiges Nutzen von manuellen Instrumenten bzw. Werkzeugen und robotisch geführten Instrumenten bzw. Werkzeugen nicht oder nur schwer möglich ist, da das Eingabegerät z.B. nicht in der Nähe des Arbeitsplatzes des Manipulators insbesondere eines Operationstischs ist oder die Orientierung des Eingabegeräts nicht an den Aufstellungsort angepasst werden kann.

Weiter haben die oben genannten Systeme den Nachteil, dass sie keinen Modus zur Schwerkraftkompensation bzw. zur sensitiven Handführung bereitstellen.

Im Hinblick auf die oben erläuterten Systeme ist es das Ziel der vorliegenden Erfindung, ein Verfahren und ein System bereitzustellen, welches eine einfache Ausrichtung eines mehrachsigen Manipulators mit einem Eingabegerät erlaubt. Dabei wird dem Bediener des Robotersystems, insbesondere während eines Arbeitsprozesses, z.B. einer Operation, ermöglicht, die Position oder Orientierung des Eingabegerätes intraoperativ zu verändern, ohne dass auf eine möglichst intuitive Steuerung verzichtet werden muss.

Diese und weitere Aufgaben werden durch den Gegenstand der Hauptansprüche gelöst.

### 3. Inhalt der Erfindung

Die vorliegende Erfindung umfasst ein Verfahren zur Ausrichtung eines mehrachsigen Manipulators mit einem zugehörigen Eingabegerät und insbesondere ein Ausrichten des Referenz-Koordinatensystems eines Eingabegeräts zum Koordinatensystem eines damit zu bedienenden Manipulators, insbesondere des Werkzeug-Koordinatensystems. Vorzugsweise handelt es sich bei dem mehrachsigen Manipulator um einen Gelenkarmroboter. Das Eingabegerät dient dabei der Steuerung des Manipulators. Eine Ausrichtung von Eingabegerät und Manipulator ist beispielsweise dann erforderlich und vorteilhaft, wenn der Bediener die Position bzw. Orientierung des Eingabegeräts ändert, um z.B. einen besseren Zugang zum Arbeitsbereich des Manipulators zu haben, wie etwa einen besseren Zugang zu einem Patienten bei einer roboterunterstützten Operation. Durch diese (Neu-)Ausrichtung von Eingabegerät und Manipulator wird es möglich, die Bedienung nach den Bedürfnissen des Bedieners einzustellen. Damit wird vorteilhafterweise eine einfachere und intuitivere Bedienung des Manipulators möglich.

In dem erfindungsgemäßen Verfahren werden eine oder mehrere Referenzbewegungen mit dem Eingabegerät ausgeführt. Dabei wird das Eingabegerät (wie etwa ein Space-Controller, berührungsempfindliche Oberflächen, wie z.B. Touchpads oder -monitore) in bestimmten Richtungen bewegt, um z.B. X- und Y-Ausrichtung des Eingabegeräts neu zu kalibrieren.

In einem weiteren Schritt werden eine oder mehrere Referenzbewegungen mit dem Manipulator ausgeführt. Dabei wird der Manipulator in bestimmte Richtungen bewegt und vorzugsweise derart, dass die Referenzbewegungen des Manipulators den mit dem Eingabegerät durchgeführten Referenzbewegungen entsprechen. Der Bediener führt also vorzugsweise die Referenzbewegungen des Manipulators so aus, dass er eine für ihn gewünschte Ausrichtung von Manipulator und Eingabegerät erreicht. Dabei definiert der Bediener vorzugsweise die Referenz-Koordinatensysteme (R-KOS) von Manipulator und Eingabegerät, indem er entsprechende Referenzbewegungen mit Manipulator und Eingabegerät ausführt. Die Referenzbewegungen werden von der Robotersteuerung erfasst.

Die vom Bediener ausgeführten Referenzbewegungen sind dabei vorzugweise geeignet, um eine Ausrichtung der beiden Referenz-Koordinatensysteme berechnen zu können. Für Anwendungen, die keine absolute Positionierung des z.B. Werkzeugs erfordern, ist es ausreichend, nur die Orientierung der beiden Referenz-Koordinatensysteme zu definieren. Eine Definition des Koordinatenursprungs (KOU) ist in diesem Fall nicht zwingend notwendig.

Durch die optionale Vorgabe eines Koordinatenursprungs kann ein Referenz-Koordinatensystem vollständig definiert werden. Eine vollständige Definition kann in Verbindung mit bildgebenden Verfahren vorteilhaft sein, beispielsweise um Patientenbilder oder -modelle in den Arbeitsraum des Manipulators abzubilden.

Ein Referenz-Koordinatensystem (und optional ein Koordinatenursprung) kann vorzugsweise mit folgenden Eingaben definiert werden:
a) mittels dreier Punkte, wobei optional einer dieser Punkte als Koordinatenursprung definiert werden kann;
b) mittels zweier Achsen, wobei optional ein Koordinatenursprung zusätzlich vorgegeben werden kann, durch:
   i. Bewegungen entlang zweier im Wesentlichen orthogonaler Geraden, die ansonsten beliebig im Raum ausgerichtet sein können;
   ii. Eine im Wesentlichen L-förmige Bewegung, d.h. ein Kurvenzug aus zwei orthogonalen Strecken;
   iii. Zwei Flächenachsen, wobei aus zwei kreisförmigen Bewegungen jeweils die zur Bewegung orthogonalen Achsen ermittelt werden, wobei die Flächenachsen alternativ auch durch zwei mit dem Manipulator bzw. dem Eingabegerät ausgeführte Drehungen bestimmt werden können; oder
   iv. einen Punkt und zwei Richtungen oder zwei Punkte und zwei Richtungen;
c) Aufspannen einer Ebene, wobei optional ein Koordinatenursprung zusätzlich vorgegeben werden kann, durch
   i. eine im Wesentlichen L-förmige Bewegung
   ii. eine im Wesentlichen kreisförmige Bewegung; oder
   iii. eine allgemeine, im Wesentlichen planare Bewegung.

Die ausgeführten Referenzbewegungen werden z.B. durch eine Steuereinrichtung erfasst, d.h. es werden sowohl die Referenzbewegungen, die mit dem Eingabegerät ausgeführt werden, als auch die Referenzbewegungen, die mit dem Manipulator ausgeführt werden, erfasst. Vorzugsweise ist es die Steuereinrichtung des Manipulators, die die ausgeführten Referenzbewegungen erfasst.

Die Bewegungen werden vorzugsweise jeweils mit einer in Manipulator bzw. Eingabegerät integrierten Positionssensorik erfasst und vorzugsweise zur Weiterverarbeitung an die Steuereinrichtung übermittelt und dort zur Weiterverarbeitung zumindest temporär gespeichert. Die Erfassung der Bewegungen kann aber auch durch ein sich um den Arbeitsraum des Manipulators und Eingabegeräts befindliches, externes Navigationssystem durchgeführt werden. Dabei werden z.B. zu erfassende Markierungen auf dem Manipulator bzw. auf dem Eingabegerät angebracht und durch das externe Navigationssystem, z.B. optisch erfasst.

In einem weiteren Schritt wird eine Transformationsmatrix basierend auf den erfassten Referenzbewegungen berechnet. Die Transformationsmatrix enthält auf Grundlage der Referenzbewegungen errechnete Werte, die der Ausrichtung des Koordinatensystems des Eingabegeräts mit dem Koordinatensystem des Manipulators dienen. Dabei werden die Koordinatensysteme von Eingabegerät und Manipulator bzw. des Werkzeugs, welches an dem Manipulator angebracht ist, aufeinander abgestimmt. Die Transformationsmatrix wird vorzugsweise auf Basis eines oder mehrerer Algorithmen berechnet, die in der Steuereinrichtung gespeichert sind.

Nach Berechnung der Transformationsmatrix erhält der Bediener vorzugsweise eine Rückmeldung von der Steuereinrichtung, z.B. optisch auf einem Monitor oder akustisch.

In einem weiteren Schritt wird die berechnete Transformationsmatrix zur Ausrichtung der Bewegungen des Eingabegeräts mit dem Manipulator angewendet. Dazu werden vorzugsweise die Bewegungen des Eingabegeräts mit Hilfe der Transformationsmatrix auf den Manipulator abgebildet. Nach der Ausrichtung kann der Manipulator wieder intuitiv über das Eingabegerät gesteuert werden.

Die Erfindung bietet den Vorteil einer verbesserten, intuitiveren und flexibleren Bedienung des Manipulators. Des Weiteren erlaubt das Verfahren, dass der Bediener das Referenz-Koordinatensystem eines Eingabegeräts in einfacher Weise zum (Werkzeug-)Koordinatensystem eines Manipulators ausrichten kann und damit eine flexible und schnelle bedienerabhängige Anpassung des Manipulators vorgenommen werden kann.

Vorzugsweise wird zwischen den Schritten des Erfassens der Referenzbewegungen und dem Berechnen der Transformationsmatrix eine Ausgleichsgeometrie für die erfassten Referenzbewegungen durch eine Steuereinrichtung berechnet. Dazu werden für die in einer Gesamt-Punktewolke erfassten Punkte der Referenzbewegungen entsprechende Teil-Punktewolken für die einzelnen Geraden und/oder Ebenen identifiziert und abgespeichert. Danach werden in die Teil-Punktewolken Geraden bzw. Ebenen eingepasst. Dies geschieht in der Regel mit einer Ausgleichsrechnung, üblicherweise nach der Methode der kleinsten Fehlerquadrate. Beispielsweise handelt es sich bei der Ausgleichgeometrie um eine Gerade, die aufgrund der (nicht zwingend geraden) Referenzbewegung berechnet wird. Eine Berechnung einer Ausgleichsgeometrie kann deshalb erforderlich sein, weil z.B. die vom Bediener ausgeführten Referenzbewegungen des Manipulators typischerweise Abweichungen von einer theoretischen Idealgeometrie aufweisen, beispielsweise wenn der Bediener beim Ausführen der Referenzbewegung des Manipulators diesen unmittelbar per Hand führt. Weiter wird vorzugsweise nach Berechnung der Ausgleichsgeometrie eine Plausibilitätsprüfung anhand von Daten der Ausgleichsgeometrie vorgenommen. Dabei werden die durch die Ausgleichsgeometrien repräsentierten Daten auf Plausibilität geprüft. Neben der Prüfung auf Vollständigkeit und Konsistenz kann die Plausibilitätsprüfung insbesondere Prüfungen auf Orthogonalität der Ausgleichsgeometrien und/oder Geradheit der Referenzbewegungen (Linie) bzw. Ebenheit der Referenzbewegung (Ebene) umfassen. Für die Beurteilung der Geradheit der Referenzbewegungen (Linie) bzw. Ebenheit der Referenzbewegung (Ebene) kann ein Gütemaß verwendet werden, das auf einem Vergleich der (gemessenen) Teil-Punktewolken mit den (berechneten) Ausgleichgeometrien basiert, beispielsweise einer mittleren oder maximalen Abweichung eines Messpunkts von der berechneten Geometrie. Für alle oben genannten Prüfschritte der Plausibilitätsprüfung werden in der Steuereinrichtung entsprechende Grenzwerte gespeichert, die optional anwendungsspezifisch definiert sein können.

Des Weiteren weist der Manipulator vorzugsweise eine oder mehrere Achsen mit Mitteln zur Erfassung von Kräften und/oder Drehmomenten auf. Diese Mittel können beispielsweise einen oder mehrere Sensoren, die vorzugsweise in einem oder mehreren Gelenken des Manipulators angeordnet sind, umfassen. Dieser eine oder die mehreren Sensoren können auch beispielsweise am Endeffektor, Manipulatorsockel oder an einer anderen Stelle entlang des Manipulators angeordnet sein. Die Mittel zur Erfassung von Kräften und/oder Drehmomenten können auch auf einer Messung von Stromwerten, vorzugsweise in den Antrieben des Manipulators, basieren. In einem Beispiel kann ein mehrachsiger Kraft-/Momentensensor am Endeffektor bereitgestellt sein, ohne dass weitere Kraft-/Momentensensoren in den Gelenken bereitgestellt werden.

Vorzugsweise können auch Mittel zur Erkennung einer Benutzerinteraktion, z.B. berührungsempfindliche Sensoren, auf dem Manipulator angeordnet sein.

Des Weiteren wird der Manipulator vorzugsweise anhand seines Werkzeugkoordinatensystems gesteuert.

Vorzugsweise umfasst der Schritt des Ausführens einer oder mehrerer Referenzbewegungen mit dem Manipulator ein unmittelbares Führen des Manipulators von Hand. Dazu wird der Manipulator durch den Bediener ergriffen bzw. gepackt und dann von Hand unmittelbar geführt, d.h. der Manipulator wird nicht mittelbar über Befehlstasten o.ä. geführt. Das unmittelbare Führen des Manipulators von Hand hat den Vorteil, dass der Bediener den Manipulator einfach greifen kann und seinen Vorstellungen entsprechend bewegen kann, damit der Manipulator genau das tut, was der Bediener ihm vorgibt. Dies ist insbesondere vorteilhaft, da es dem Bediener einfach und ohne große Umstände ermöglicht, beispielweise während eines Arbeitsprozesses, das Eingabegerät optimal und intuitiv mit dem Manipulator auszurichten. Dies führt zu einer Entlastung des Bedieners, da ihm umständliche Eingaben an Geräten und Ähnlichem zur optimalen Ausrichtung des Eingabegeräts erspart bleiben und kein vertieftes technisches Wissen über die Funktionsweise des Robotersystems erforderlich ist. Das erfindungsgemäße Verfahren ist des Weiteren dazu geeignet, mit einem Manipulator mit darauf angeordneten Sensoren, insbesondere integrierten Momenten-Sensoren, durchgeführt zu werden, da diese eine unmittelbare Führung des Manipulators von Hand in einfacher Weise erlauben. Vorzugsweise umfasst der Schritt des Ausführens einer oder mehrerer Referenzbewegungen mit dem Manipulator daher den Betrieb des Manipulators in einem Betriebsmodus zur aktiven Nachgiebigkeitsregelung, insbesondere Schwerkraftkompensation.

Der Manipulator ist vorzugsweise mit einem oder mehreren Werkzeugen ausgestattet, so dass diese durch den Manipulator bewegt werden können. Insbesondere können diese Werkzeuge medizinische Instrumente sein, wobei die medizinischen Instrumente Endoskope, Ultraschallköpfe, Instrumente für die offene oder minimal-invasive Chirurgie, Punktionskanülen oder Biopsienadeln umfassen. In einer weiteren Ausführungsform können diese Werkzeuge insbesondere auch industriell verwendete Bearbeitungswerkzeuge z.B. zum Fügen, Kleben, Schweißen, Falzen, Bohren, Schrauben etc. sein.

Vorzugsweise umfasst das Eingabegerät einen haptischen Handcontroller, Joystick, 3-D Motion Controller, kapazitives Touchpad und/oder mehrere Schalter zum Auslösen spezieller Funktionen und/oder vorprogrammierter Bewegungssequenzen des Manipulators und/oder des Werkzeugs.

Vorzugsweise ist das Eingabegerät, beispielsweise lösbar, an einem gelenkigen Haltearm angebracht. Damit wird es möglich, das Eingabegerät flexibel zu einer neuen Position zu bewegen und, wenn nötig, auch von seinem aktuellen Anbringungsort zu lösen und an einem anderen Ort anzubringen. Vorzugsweise weisen die Gelenke des Haltearms vorzugsweise Sensorik zur Erfassung der Gelenkstellungen des Haltearms auf. Damit kann die Steuereinrichtung zu jedem Zeitpunkt bestimmen, in welcher Stellung sich das Eingabegerät befindet. Diese Messwerte können dann genutzt werden, die Transformationsmatrix nur einmal zu Beginn eines Arbeitsprozesses nach dem oben beschriebenen erfindungsgemäßen Verfahren zu ermitteln. Bei einem Verstellen des Haltearms durch den Bediener aus der ursprünglichen Stellung wird die Transformationsmatrix in der Steuereinrichtung automatisch anhand der gemessenen Positionswerte aktualisiert. Optional erhält der Bediener nach erfolgreicher Aktualisierung der Transformationsmatrix eine Rückmeldung von der Steuereinrichtung (z.B. optisch auf Monitor, durch eine Signallampe und/oder akustisch).

Vorzugsweise werden die Referenzbewegungen von Manipulator und Eingabegerät simultan oder sequenziell erfasst. Zur Aufnahme der Referenzbewegungen ist die Steuereinrichtung z.B. so konfiguriert, dass die vom Bediener ausgeführten Referenzbewegungen von Manipulator und Eingabegerät simultan erfasst werden. Das bedeutet im vorliegenden Fall, dass der Bediener die erforderlichen Bewegungen (eine oder mehrere Referenzbewegungen) gleichzeitig mit dem Manipulator und dem Eingabegerät ausführt. Beispielsweise führt der Bediener mit der linken Hand die Bewegung mit dem Manipulator und mit der rechten Hand mit dem Eingabegerät aus (oder umgekehrt). Alternativ ist es ergebnisgleich möglich, dass der Bediener alle erforderlichen Bewegungen nacheinander (sequentiell) mit dem Manipulator und dem Eingabegerät ausführt. Das bedeutet, dass der Bediener zuerst die Bewegung mit dem Eingabegerät und anschließend mit dem Manipulator ausführt (oder umgekehrt).

In einem anderen Beispiel ist es ergebnisgleich möglich, dass der Bediener zunächst sämtliche Referenzbewegungen an entweder dem Eingabegerät oder am Manipulator ausführt und danach sämtliche entsprechenden Referenzbewegungen an dem jeweils anderen Gerät (Manipulator oder Eingabegerät), je nachdem, ob der Bediener an dem Eingabegerät oder Manipulator begonnen hat.

Des Weiteren sind vorzugsweise ein oder mehrere externe Bedienelemente, insbesondere ein mit der Steuereinrichtung verbundener Fußschalter, eingerichtet zum Umschalten zwischen Betriebsmodi oder zum Auslösen einer Aktion des Werkzeugs. Diese Betriebsmodi umfassen beispielsweise einen Ausrichtemodus, der zum Ausführen der Ausrichtung des Eingabegeräts mit dem mehrachsigen Manipulator aktiviert werden kann.

Vorzugsweise ist ein externer Monitor vorgesehen, der eingerichtet ist zum Darstellen von Kamerabildern, z.B. Endoskopbildern und/oder Aufnahmen eines externen Bildgebungs- und/oder Navigationssystems.

Erfindungsgemäß wird weiter ein Robotersystem, insbesondere ein modulares Robotersystem, bereitgestellt, welches eine

Steuereinrichtung, einen Manipulator und ein Eingabegerät umfasst. Vorzugsweise ist die Steuereinrichtung eingerichtet, eine Ausrichtung des Manipulators mit dem Eingabegerät zu erlauben, indem sie ausgeführte Referenzbewegungen des Eingabegeräts und des Manipulators erfasst, eine Transformationsmatrix basierend auf den erfassten Referenzbewegungen berechnet und die berechnete Transformationsmatrix zur Ausrichtung der Bewegungen des Eingabegeräts auf den Manipulator anwendet.

Des Weiteren wird erfindungsgemäß eine Verwendung eines oben beschriebenen Verfahrens während einer Operation an einem Menschen oder in einem industriellen Prozess bereitgestellt.

Die (Neu-)Ausrichtung des Manipulators mit dem Eingabegerät kann beispielsweise durch Auswählen eines Ausrichtemodus eingeleitet werden. Dieser Ausrichtemodus kann entweder unmittelbar nach dem Einschalten oder Zurücksetzen des Manipulators bzw. der Robotersteuerung aktiv sein oder kann durch eine Benutzereingabe oder Programmsequenz aktiviert werden.

Vorzugsweise kann eine vom Bediener ausgeführte Referenzbewegung dazu verwendet werden, eine (grundlegende) Vorgabe für eine Bewegungsskalierung zwischen Eingabegerät und Manipulator zu bestimmen. Dafür können entweder die erfassten Referenzbewegungen verwendet werden oder separate Bewegungen erfasst werden. Ausschlaggebend für die Skalierung ist in beiden Fällen das Verhältnis der für die Referenzbewegung genutzten Bewegungsräume von Eingabegerät und Manipulator.

### 4. Ausführungsbeispiel

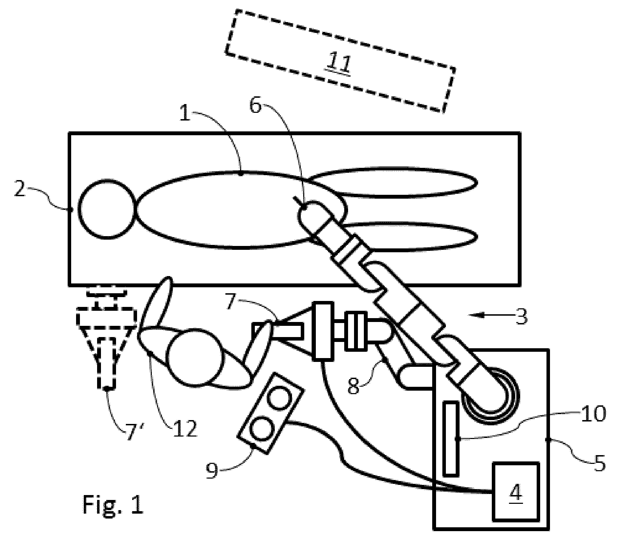

Im Folgenden wird die vorliegende Erfindung anhand der beiliegenden Figur näher beschrieben. Das erfindungsgemäße Verfahren eignet sich insbesondere für den Einsatz bei einem modularen Robotersystem, wie es in Figur 1 dargestellt ist.

Ein Patient 1 liegt auf einem Untersuchungs- oder Operationstisch 2. Ein Manipulator 3 ist zusammen mit einer zugehörigen Steuereinrichtung 4 in einen fahrbaren Wagen 5 integriert. Am distalen Ende des Manipulators 3 ist ein medizinisches Instrument 6 so angebracht, dass es durch den Manipulator 3 bewegt werden kann. Weiterhin ist an dem Wagen 5 ein Eingabegerät 7 angebracht, das mit der Steuereinrichtung 4 verbunden ist, damit ein Bediener die Bewegung des Manipulators 3 steuern kann. Der Bediener kann das Eingabegerät 7 mit einem gelenkigen Haltearm 8 ergonomisch in seinem Arbeitsumfeld ausrichten. Am Wagen ist starr oder gelenkig ein Display 10 angebracht, das optional berührungsempfindlich sein kann (z.B. Touchscreen), zur Darstellung von Betriebsparametern und/oder des Systemzustands.

Das Robotersystem wird vom medizinischen Personal bzw. Bediener so an dem Tisch 2 platziert, dass der Eingriffs- bzw. Untersuchungsort am Patienten 1 mit dem medizinischen Instrument 6 bei gleichzeitig hoher Manipulierbarkeit des Manipulators 3 erreicht wird und der Patient 1 weiterhin für den Bediener 12 und optional zusätzliches medizinisches Personal ausreichend zugänglich ist. Vor Durchführung eines Eingriffs, einer Operation bzw. einer Untersuchung, wird das Eingabegerät 7 vom Bediener in einer für ihn komfortablen Position mit Hilfe des Haltearms 8 ausgerichtet bzw. bewegt. Dabei kann das Eingabegerät 7 von dem Haltearm 8 optional gelöst werden und an einem anderen Gegenstand, beispielsweise an dem Tisch 2, befestigt werden. Diese Anbringungsmöglichkeit ist in Figur 1 durch Eingabegerät 7' dargestellt.

Da sich das Eingabegerät vorher in einer bestimmten Ausrichtung zum Manipulator befunden hat und der Bediener nun das Eingabegerät 7 z.B. mit Hilfe des Haltearms 8 verstellt hat, wird der Bediener nun nicht mehr unbedingt das Robotersystem intuitiv für sich nutzen können, da Eingabegerät 7 und Manipulator 3 nicht mehr optimal miteinander ausgerichtet sind. Um Eingabegerät 7 und Manipulator 3 auszurichten, aktiviert der Bediener zunächst einen Modus zur Ausrichtung der Koordinatensysteme von Eingabegerät 7 und Manipulator 3 (Ausrichtemodus). Dieser Modus ist entweder unmittelbar nach dem Einschalten oder Zurücksetzen (Reset) des Robotersystems bzw. der Steuereinrichtung aktiv oder wird durch eine Benutzereingabe oder Programmsequenz aktiviert. Letzteres erfolgt beispielsweise mittels Bedienelement 10, Monitor 11 (sofern berührungsempfindlich) oder einem anderem, in Wagen 5 integrierten, Bedienelement. Durch Aktivierung des Ausrichtemodus wird der Manipulator 3 in einem Betriebsmodus zur aktiven Nachgiebigkeitsregelung, insbesondere Schwerkraftkompensation betrieben, sodass der Bediener 12 diesen frei bewegen kann.

Der Bediener führt zunächst eine Referenzbewegung mit dem Eingabegerät 7 aus und danach eine entsprechende Referenzbewegung mit dem Manipulator 3. Nach Ausführung der Referenzbewegungen, erfasst die Steuereinrichtung 4 diese Referenzbewegungen, berechnet daraus eine Transformationsmatrix und wendet diese zur Ausrichtung von Eingabegerät 7 und Manipulator 3 an. Damit hat der Bediener auf sehr einfache und intuitive Weise eine für ihn optimale (Neu-)Ausrichtung von Eingabegerät 7 und Manipulator 3 erreicht, was ihm eine intuitive Anwendung des Robotersystems während der Operation ermöglicht. Weiterhin wird dem Bediener ein gleichzeitiges Verwenden des Robotersystems und manuell bedientem Instrument ermöglicht, da der Bediener sich mit dem Robotersystem am Patienten befindet.

## Patentansprüche

1. Verfahren zur Ausrichtung eines mehrachsigen Manipulators (3) mit einem Eingabegerät (7), welches der Steuerung des Manipulators (3) dient, aufweisend die folgenden Schritte:
a) Ausführen einer oder mehrerer Referenzbewegungen mit dem Eingabegerät (7);
b) Ausführen einer oder mehrerer Referenzbewegungen mit dem Manipulator (3);
c) Erfassen der ausgeführten Referenzbewegungen;
d) Berechnen einer Transformationsmatrix basierend auf den erfassten Referenzbewegungen; und
e) Anwenden der berechneten Transformationsmatrix zur Ausrichtung der Bewegungen des Eingabegeräts (7) mit dem Manipulator (3).

2. Verfahren nach Anspruch 1, wobei zwischen den Schritten c) und d) eine Ausgleichsgeometrie für die erfassten Referenzbewegungen durch eine Steuereinrichtung (4) berechnet wird; und
wobei nach Berechnung der Ausgleichsgeometrie eine Plausibilitätsprüfung anhand von Daten der Ausgleichsgeometrie vorgenommen wird.

3. Verfahren nach einem der vorhergehenden Ansprüche, wobei der Manipulator (3) eine oder mehrere Achsen mit Mitteln, insbesondere Sensoren, zur Erfassung von Kräften und Drehmomenten aufweist.

4. Verfahren nach einem der Ansprüche 1 bis 2, wobei der Manipulator (3) eine oder mehrere Achsen mit Mitteln zur Erfassung einer Benutzerinteraktion aufweist.

5. Verfahren nach einem der vorhergehenden Ansprüche, wobei der Manipulator (3) ein Werkzeugkoordinatensystem aufweist und anhand seines Werkzeugkoordinatensystems gesteuert wird.

6. Verfahren nach einem der vorhergehenden Ansprüche, wobei der Schritt des Ausführens einer oder mehrerer Referenzbewegungen mit dem Manipulator (3) ein unmittelbares Führen des Manipulators (3) von Hand umfasst und den Betrieb des Manipulators (3) in einem Betriebsmodus zur aktiven Nachgiebigkeitsregelung umfasst.

7. Verfahren nach einem der vorhergehenden Ansprüche, wobei der Manipulator (3) mit zumindest einem Werkzeug, insbesondere einem medizinischen Instrument (6), ausgestattet ist, so dass dieses durch den Manipulator (3) bewegt werden kann.

8. Verfahren nach Anspruch 7, wobei das zumindest eine Werkzeug ein industriell verwendetes Bearbeitungswerkzeug zum Fügen, Kleben, Schweißen, Falzen, Bohren, oder Schrauben ist.

9. Verfahren nach einem der vorhergehenden Ansprüche, wobei das Eingabegerät (7) eines der Folgenden umfasst: haptischer Handcontroller, Joystick, 3-D Motion Controller, kapazitives Touchpad, Schalter zum Auslösen spezieller Funktionen oder vorprogrammierter Bewegungssequenzen des Manipulators (3), berührungslose Sensoren, wie Kinect-Kamera oder Leap-Device.

10. Verfahren nach einem der vorhergehenden Ansprüche, wobei das Eingabegerät (7), vorzugsweise lösbar, an einem gelenkigen Haltearm (8) angebracht ist; und
wobei die Gelenke des Haltearms (8) Sensorik aufweisen zur Erfassung der Gelenkstellungen des Haltearms (8).

11. Verfahren nach einem der Ansprüche 1 bis 9, wobei das Eingabegerät (7), vorzugsweise lösbar, an einem gelenkigen Haltearm (8) angebracht ist; und
wobei die Lage des Haltearms (8) und/oder des Eingabegeräts (7) mit einem externen Navigationssystem erfasst wird.

12. Verfahren nach einem der vorhergehenden Ansprüche, wobei die Referenzbewegungen von Manipulator (3) und Eingabegerät (7) simultan oder sequenziell erfasst werden.

13. Verfahren nach einem der vorhergehenden Ansprüche, wobei ein oder mehrere externe Bedienelemente (9), insbesondere ein mit der Steuereinrichtung (4) verbundener Fußschalter, eingerichtet sind zum:
Umschalten zwischen Betriebsmodi oder zum Auslösen einer Aktion des Werkzeugs; und
wobei ein externer Monitor (11) eingerichtet ist zum:
Darstellen von Aufnahmen eines Kamerasystems, insbesondere einer Endoskopkamera und/oder externen Bildgebungs- und/oder Navigationssystems.

14. Robotersystem, insbesondere ein modulares Robotersystem, umfassend eine Steuereinrichtung (4), einen Manipulator (3) und ein Eingabegerät (7),
wobei die Steuereinrichtung (4) eingerichtet ist eine Ausrichtung des Manipulators (3) mit dem Eingabegerät (7) zu erlauben durch:
Erfassen von ausgeführten Referenzbewegungen des Eingabegeräts (7) und des Manipulators (3);
Berechnen einer Transformationsmatrix basierend auf den erfassten Referenzbewegungen; und
Anwenden der berechneten Transformationsmatrix zur Ausrichtung der Bewegungen des Eingabegeräts (7) auf den Manipulator (3).

15. Verwendung eines Verfahrens gemäß einem der Ansprüche 1 bis 13 während einer Operation an einem Menschen oder in einem industriellen Prozess.
